# EUROPÄISCHE PATENTANMELDUNG

(11) **EP 0 627 236 A2**
(43) Veröffentlichungstag der Anmeldung: **07.12.1994**
(21) Anmeldenummer: 94105991.7
(22) Anmeldetag: 18.04.1994
(51) Int. Cl.: A61N 1/16

(54) **Strahlenschutzvorrichtung**

(30) Priorität: 03.06.1993 DE 9308355 U
(71) Anmelder: TERRA BOHN GmbH, D-88085 Langenargen (DE)
(72) Erfinder: Bohn, Christine, D-88085 Langenargen (DE)
(74) Vertreter: Geyer, Ulrich F., Dr. Dipl.-Phys.

(57) **Zusammenfassung**

Bei einer Strahlenschutzvorrichtung ergibt sich eine bessere Stahlenschutzwirkung durch wenigstens ein elektromagnetisch schwingendes Gebilde. Die Erfindung ist insbesondere im Zusammenhang mit einer Strahlenschutzdecke vorteilhaft, die aus wenigstens zwei Grundteilen mit jeweils mindestens einem schwingendem Gebilde besteht.

## Beschreibung

Die Erfindung betrifft eine Strahlenschutzvorrichtung.

Bekannte Strahlenschutzvorrichtungen, die insbesondere in Betten-, Liege- oder Sitzbereich und für Bodenbeläge verwendet werden, weisen Kupferlitzen auf, die auf einer Unterlage als Trägermittel befestigt sind. Derartige Strahlenschutzvorrichtungen haben sich zwar als vorteilhaft gegen Abschirmungen von Erdstrahlen und Wasseradern erwiesen, bei höherer Strahlungsintensität ist jedoch eine Auflösung der dabei entstehenden Ladung nicht mehr sichergestellt.

Der Erfindung liegt daher die Aufgabe zugrunde, eine Strahlenschutzvorrichtung zu schaffen, die die Strahlenschutzwirkung verbessert, und zwar sowohl in Hinblick auf Erdstrahlen und von Wasseradern ausgehende Strahlung, als auch hinsichtlich kosmischer Strahlung.

Die gestellte Aufgabe wird erfindungsgemäß dadurch gelöst, daß die Strahlenschutzvorrichtung wenigstens ein elektromagnetisch schwingendes Gebilde aufweist.

Auf Grund der Verwendung eines elektromagnetisch schwingenden Gebildes, beispielsweise eines Dipols oder eines Schwingkreises, in Zusammenhang mit der Strahlenschutzvorrichtung können elektrische und/oder elektromagnetische Störfelder, die beispielsweise durch Aufladungen auf Grund ionisierter Strahlung entstehen, zuverlässig verhindert bzw. aufgelöst werden. Derartige elektromagnetisch schwingende Gebilde sind insbesondere auch für den Schutz gegen Strahlungen geeignet, die von elektromagnetischen Wechselfeldern, wie angeschlossenen elektrischen Geräten oder elektrischen Leitungen herrühren.

Die erfindungsgemäße Strahlenschutzvorrichtung ist insbesondere als Schlaf-, Liege-, Sitz-und/oder Bodenmatte, als Decke oder als Bodenbelag verwendbar.

Eine besonders vorteilhafte Ausgestaltung der Erfindung besteht darin, daß die Strahlenschutzvorrichtung aus wenigstens zwei Grundteilen zusammengesetzt ist, die jeweils wenigstens ein schwingendes Gebilde aufweisen. Auf diese Weise ergibt sich eine sehr einfache Handhabung und Fertigung der Strahlenschutzvorrichtung für die verschiedenen Verwendungsformen, indem eine entsprechende Anzahl von Grundteilen zu einer vollständigen Strahlenschutzvorrichtung zusammengesetzt wird. Es ist daher nicht mehr erforderlich, große Strahlenschutzvorrichtungen nur wegen ein oder zwei schwingenden Gebilden zu fertigen.

Besonders vorteilhaft vorteilhaft ist es, wenn die Grundteile Klettverschlüsse aufweisen. Es ist jedoch auch möglich, zur Verbindung der Grundteile Druckknöpfe vorzusehen.

Gemäß einer besonders vorteilhaften Ausführungsform ist jedes Grundteil rechteckförmig ausgebildet, wobei das Rechteck eine lange Seitenkante a von 800 bis 1200 mm und eine kurze Seitenkante b von 400 bis 600 mm aufweist. Derartige Grundteile können dann zu verschiedenen Strahlenschutz Vorrichtungen mit unterschiedlicher Anwendungsart zusammengesetzt werden. Vorzugsweise ist dabei für einen Arbeitsplatz nur ein Grundteil vorgesehen, während für einen Sitzplatz zwei Grundteile und einen Bettenplatz drei oder vier Grundteile vorgesehen sind. Durch die Verbindung dieser Grundteile ergibt sich eine sehr flexible Anwendungsform, ohne daß für jeden Anwendungsfall spezielle Strahlenschutzvorrichtungen gefertigt werden müßten. Vorteilhafterweise kann ein Bodenbelag ebenfalls aus einer entsprechenden Anzahl von Grundteilen ähnlich der Auslegung eines Raumes mit Tatami-Matten gebildet werden.

Gemäß einer besonders vorteilhaften Ausgestaltung der Erfindung ist das elektromagnetisch schwingende Gebilde ein Dipol. Als vorteilhafte alternative Ausgestaltung kann das schwingende Gebilde jedoch auch ein Schwingkreis sein.

Vorzugsweise weist das Material, aus dem das schwingende Gebilde, insbesondere der Dipol, besteht, eine hohe elektrische Leitfähigkeit auf.

Vorzugsweise weisen die Dipole jeweils eine Schlingenform auf, die aus zwei vorzugsweise kreisförmig gebogenen Haken besteht. Besonders vorteilhaft ist es, wenn zwischen den einander gegenüberliegenden Enden der Schlinge ein die Haken verbindender Steg vorgesehen ist.

Die Schwingkreise bzw. die Dipole bestehen vorzugsweise aus einem Bundmetall, insbesondere aus einer Kupferlitze mit kreisförmigem oder quadratischem Querschnitt. Bei Verwendung eines Dipols als elektromagnetisch schwingendem Gebilde ist es vorteilhaft, wenn der Abstand zwischen den Enden der Haken und dem Verbindungssteg jeweils ein Mehrfaches der Materialstärke der Dipole beträgt.

Die Anordnung der Dipole ist vorteilhafterweise so gewählt, daß sie - vorzugsweise gleichmäßig verteilt - ganz oder teilweise mit einem Baumwollgewebe und/oder einem Zellstoffgewebe ein- oder mehrfach ummantelt sind, etwa dadurch, daß das Baumwollgewebe und/oder das Zellstoffgewebe in einer oder mehreren Lagen um die Dipole gewikkelt werden. Alternativ ist es jedoch auch möglich, das Baumwoll- und/oder Zellstoffgewebe jeweils als Schlauch auszubilden, der über das schwingende Gebilde gezogen wird.

Vorteilhaft ist es weiterhin, wenn die Enden der mit dem Baumwoll- und/oder Zellstoffgewebe ummantelten Haken der schwingenden Gebilde, insbesondere der Dipole, etwa in einer der Metallstärke entsprechenden Länge freigehalten sind.

Vorzugsweise sind die schwingenden Gebilde, etwa die Dipole, auf einer Schaumstoffunterlage, einer Kunststoff-Folie, einer Gummimatte, einer Bodenmatte oder dgl., als Trägermittel aufgebracht, beispielsweise aufgeklebt oder eingelegt. Im Falle eines Dipols als schwingendes Gebilde sind die Enden der Haken ein- oder beidseitig durch einen vorzugsweise doppelseitigen Klebestreifen fixiert.

Eine weitere vorteilhafte Ausgestaltung der Erfindung besteht darin, daß die schwingenden Gebilde zwischen zwei Lagen eines Trägermaterials angeordnet sind, das beispielsweise aus Schaumstoff, Kunststoff oder Gummi besteht. Das Material kann dabei auch eine Kunststoff-Folie sein.

Vorzugsweise sind die schwingenden Gebilde, etwa die Dipole, hinsichtlich ihrer Abmessungen derart ausgebildet, daß sie auf dem Trägermittel gleichmäßig verteilt angeordnet sind. Vorteilhaft ist es weiterhin, daß der seitliche Abstand zwischen den schwingenden Gebilden und der Abstand der schwingenden Gebilde zu den Außenkanten des Trägermaterials etwa gleich groß ist.

Sehr vorteilhaft ist es im Zusammenhang mit den Grundteilen, wenn jeweils jedes Grundteil ein elektrisch schwingendes Gebildet aufweist, das bezüglich der aufeinander senkrecht stehenden Mittelachsen des Grundteils im wesentlichen symmetrisch angeordnet sind. Bei Zusammensetzen einer Strahlenschutzvorrlchtung durch wenigstens zwei Grundteile ergibt sich dadurch eine gleichmäßige Beabstandung der schwingenden Gebilde zueinander, ohne daß bei der Fertigung besondere Maßnahmen hierfür getroffen zu werden brauchen.

Die erfindungsgemäße Strahlenschutzvorrichtung führt zu einem zuverlässigen Schutz vor Erdstrahlen, vor von Wasseradern ausgehenden Strahlen sowie vor kosmischen Strahlen und darüber hinaus vor Global-Gitternetzstrahlung. Bei der erfindungsgemäßen Strahlenschutzvorrichtung findet eine Aufladung nicht statt, so daß eine gleichmäßig hohe Schutzwirkung auch über lange Zeiträume hinweg sichergestellt ist. Die elektromagnetisch schwingenden Gebilde, insbesondere ein Dipol, der vorzugsweise schlingenförmig gebogen ist, bilden einen Schutzschild, durch den den Erdstrahlen sowie von Wasseradern ausgehende Strahlen neutralisiert und kosmische Strahlen abgelegt wird.

Weiterhin ist mit der erfindungsgemäßen Strahlenschutzvorrichtung eine Umfeld-Entstrahlung erreichbar, so daß Strahlung, die durch Elektrogeräte, elektrische Leitung in und außerhalb des Hauses, im Estrich verlegte Rohre und dgl., entstehen, neutralisiert werden. Bei Verwendung der Strahlenschutzvorrichtung als Bodenbelag wird darüber hinaus eine elektrostatische Aufladung von Teppichböden vermieden.

Bei der Verwendung von Dioden als schwingende Gebilde ist die Ummantelung mit Baumwoll-und/oder Zellstoffgewebe besonders vorteilhaft, da dies einen besonders hohen Schutz vor Erdstrahlung und Strahlung, die von Wasseradern hervorgerufen werden, sicherstellt.

Die Art, Ausbildung und Dimensionierung der elektromagnetisch schwingenden Gebilde, beispielsweise die Art der Ausformung, der Rundung oder der Ummantelung der Dipole ermöglicht auch eine Anpassung an die Strahlung, die in dem speziellen Anwendungsfall besonders stark vorherrscht, so daß die Strahlenschutzvorrichtung durch besondere Ausführungsformen in den einzelnen Fällen eine für die spezielle Anwendungsform besonders hohen Schutzwirkung erzielbar ist.

Die Erfindung wird nachstehend anhand von bevorzugten Ausführungsbeispielen unter Bezugnahme auf die Figuren erläutert. Es zeigen:
Fig. 1 eine schematische Darstellung einer Ausführungsform der erfindungsgemäßen Strahlenschutzvorrichtung mit einzelnen Grundteilen;
Fig. 2 eine Ausführungsform, bei der in einer zweilagigen Decke zwischen beiden Lagen Dipole angeordnet sind, in schematischer, teilweise aufgebrochener Darstellung;
Fig. 3 eine schematische Querschnittsdarstellung entlang der in Fig. 2 eingezeichneten Schnittlinie 111-111 und
Fig. 4 eine schematische Querschnittsdarstellung entlang der in Fig. 2 dargestellten Schnittlinie IV-IV.

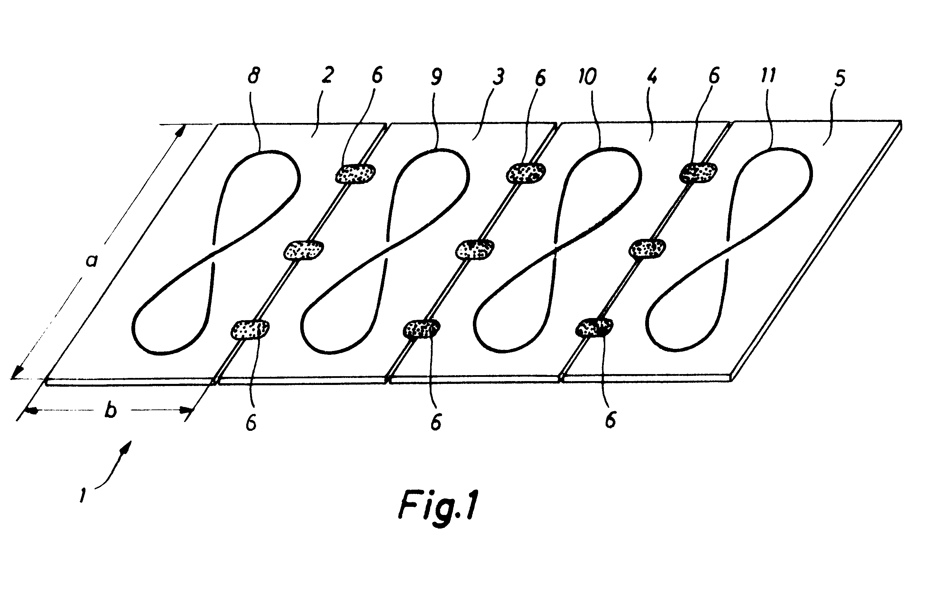

Die in Fig. 1 dargestellte Ausführungsform einer Strahlenschutzvorrichtung 1 besteht aus vier Grundteilen 2, 3, 4, 5, die jeweils mit Klettverschlüssen oder Knöpfen 6 miteinander verbunden sind.

Jedes dieser Grundteile 2, 3, 4, 5 weist ein elektromagnetisch schwingendes Gebilde, im vorliegenden Ausführungsfall jeweils einen Dipol 8, 9, 10, 11 auf, der in bzw. an den einzelnen Grundteilen 2, 3, 4, 5 so angeordnet ist, daß er im wesentlichen denselben Abstand zu den Rändern der Grundteile 2, 3, 4, 5 aufweist. Bei einer Verbindung der Grundteile 2, 3, 4, 5 miteinander ergibt sich auf diese Weise automatisch ein jeweils gleicher Abstand zwischen den Dipolen 8, 9, 10, 11 der auf diese Weise gebildeten Strahlenschutzvorrichtung.

Obgleich dies in Fig. 1 nicht dargestellt ist, kann selbstverständlich an den schmalen Seiten der Grundteile 2, 3, 4, 5 ebenfalls jeweils ein weiteres Grundteil - gegebenenfalls wiederum mittels Klettverschlüssen oder Druckknöpfen - angebracht sein, so daß die Strahlenschutzvorrichtung auch breiter als die in Fig. 1 dargestellte Vorrichtung ausgebildet werden kann.

Die Grundteile 2, 3, 4, 5 und/oder die aus den Grundteilen 2, 3, 4, 5 zusammengesetzte Strahlenschutzvorrichtung können eine beliebige Form aufweisen. Vorteilhaft ist es jedoch, die Grundteile 2, 3, 4, 5 als Rechteckteile mit einer langen Seitenkante a von vorzugsweise 900 bis 1200 mm und mit einer kurzen Seitenkante b von ca. 470 bis 500 mm auszubilden. Diese Standardmaße ermöglichen eine sehr einfache Kombination der Grundteile 2, 3, 4, 5 zu größeren Strahlenschutzvorrichtungen, etwa zu einer Decke oder einer Bettunterlage mit Abmessungen von ca. 1000 x 2000 mm. Dafür wären dann vier der die genannten Abmessungen aufweisenden Grundteile 2, 3, 4, 5 erforderlich, wie dies in Fig. 1 dargestellt ist.

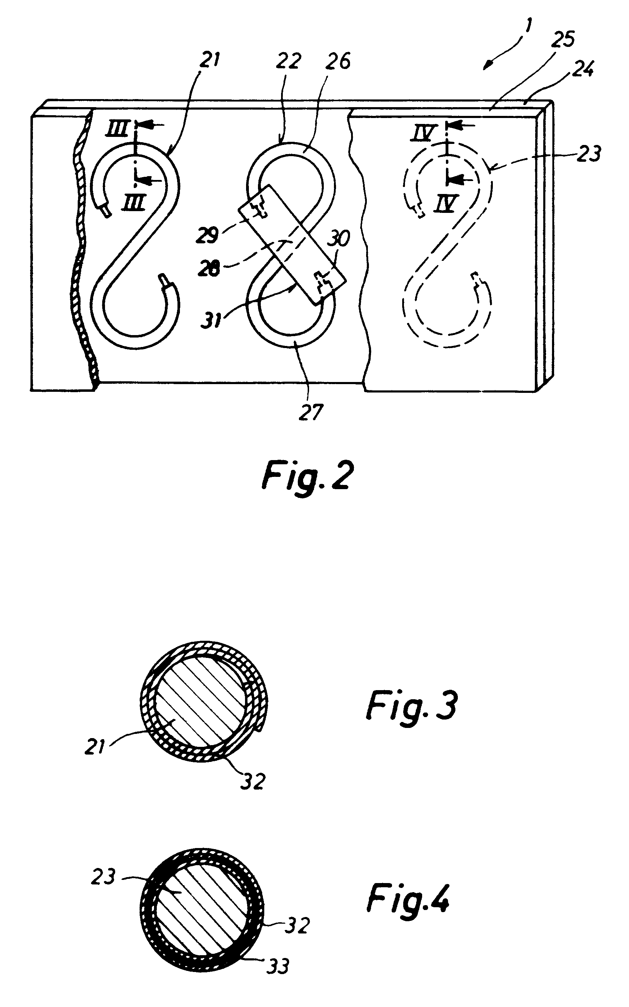

Fig. 2 zeigt eine weitere Ausführungsform der Erfindung, bei der die elektromagnetisch schwingenden Gebilde - im dargestellten Falle wiederum Dipole 21, 22, 23 in oder an einem Stück bestehenden Strahlenschutzvorrichtung 1 angebracht sind. Bei diesem Ausfüfhrungsbeispiel besteht die Strahlenschutzvorrichtung 1 aus einem Trägermaterial 24, beispielsweise aus einer Schaumstoffunterlage oder einer Kunststoff-Folie, auf die die Dipole 21, 22, 23 aufgeklebt sind, die gegebenenfalls unterschiedlich ausgeführt sein können. Die Dipole 21, 22, 23 sind mit einer weiteren Schaumstoffauflage 25 abgedeckt.

Bei der dargestellten Ausführungsform der elektromagnetisch schwingenden Gebilde in Form von Dipolen 21, 22, 23 bestehen diese aus jeweils einem Haken 26, 27, die über einen Steg 28 miteinander verbunden sind. Die Dipole 21, 22, 23, die beispielsweise aus einer Kupferfolie bestehen können, sind auf dem Trägermittel 24 aufgeklebt, so daß dadurch der Abstand zwischen den Enden 29, 30 der Haken 26, 27 fixiert ist. Das Aufheben kann beispielsweise mit einem doppelseitigen Klebestreifen erfolgen.

Die in Fig. 3 dargestellte schematische Querschnittsdarstellung entlang der in Fig. 2 eingezeichneten Schnittlinie 111-111 zeigt eine Ausführungsform der erfindungsgemäßen Strahlenschutzvorrichtung mit einem Dipol 21 als elektromagnetischem schwingendem Gebilde, der mit einem Baumwollgewebe 32 ein- oder mehrfach ummantelt ist. Zu diesem Zwecke wird - wie aus Fig. 3 ersichtlich ist - als Herstellungsmöglichkeit das Baumwollgewebe 32 um den Dipol 21 gewickelt.

Fig. 4 zeigt eine Querschnittsdarstellung entlang der in Fig. 2 eingezeichneten Schnittlinie IV-IV durch den Dipol 23. Bei dieser Ausführungsform ist das Baumwollgewebe 32 und/oder ein Zellstoffgewebe 33 jeweils als Schlauch ausgebildet und über den Dipol 23 gezogen. Vorzugsweise sind die Enden des Dipols 21 bzw. 23 freigehalten, damit keine Aufladung erfolgt.

Die Erfindung wurde anhand bevorzugter Ausführungsformen beschrieben. Dem Fachmann sind jedoch zahlreiche Abwandlungen und Ausgestaltungen möglich, ohne daß dadurch der Erfindungsgedanke verlassen wird. So können die elektromagnetisch schwingenden Gebilde, beispielsweise die Dipole 8, 9, 10, 11 bzw. 21, 22, 23 eine den entsprechenden Anwendungen angepaßte Form, Rundung und/oder Dimensionierung aufweisen. Darüber hinaus können auch das Trägermittel 24 sowie die Stoffauflage 25 der Strahlenschutzvorrichtung 1 mit einem Bezug versehen sein.

Zusammenfassend kann die Erfindung folgendermaßen beschrieben werden:
1. Strahlenschutzvorrichtung gekennzeichnet durch wenigstens ein elektromagnetisch schwingendes Gebilde (2, 3, 4, 5; 21, 22, 23).
2. Strahlenschutzvorichtung, gekennzeichnet durch die Verwendung als Schlaf-, Liege-, Sitz-und/oder Bodenmatte bzw. -belag.
3. Strahlenschutzvorrichtung, dadurch gekennzeichnet, daß die Strahlenschutzvorrichtung (1) aus wenigstens zwei Grundteilen (2, 3, 4, 5) zusammengesetzt ist, die jeweils wenigstens ein schwingendes Gebilde (8, 9, 10, 11) aufweisen (Fig. 1).
4. Strahlenschutzvorrichtung, dadruch gekennzeichnet, daß die Grundteile (2, 3, 4, 5) Klettverschlüsse (6) aufweisen (Fig. 1).
5. Strahlenschutzvorrichtung, dadurch gekennzeichnet, daß die Grundteile (2, 3, 4, 5) Druckknöpfe aufweisen.
6. Strahlenschutzvorrichtgung, dadurch gekennzeichnet, daß jedes Grundteil ein Rechteck mit einer langen Seitenkante (a) von 800 bis 1200 mm und einer kurzen Seitenkante (b) von 400 bis 600 mm ist.
7. Strahlenschutzvorrichtung, dadurch gekennzeichnet, daß für einen Arbeitsplatz ein Grundteil (2) vorgesehen ist.
8. Strahlenschutzvorrichtung, dadurch gekennzeichnet, daß für einen Sitzplatz zwei Grundteile (3, 4) vorgesehen sind.
9. Strahlenschutzvorrichtung, dadurch gekennzeichnet, daß für einen Bettenplatz drei oder vier Grundteile (2, 3, 4, 5) vorgesehen sind.
10. Strahlenschutzvorrichtung, dadurch gekennzeichnet, daß das schwingende Gebilde (8, 9, 10, 11; 21, 22, 23) ein Dipol ist.
11. Strahlenschutzvorrichtung, dadurch gekennzeichnet, daß das schwingende Gebilde (8, 9, 10, 11; 21, 22, 23) ein Schwingkreis ist.
12. Strahlenschutzvorrichtung, dadurch gekennzeichnet, daß das Material des schwingenden Gebildes (8, 9, 10, 11; 21, 22, 23) eine hohe elektrische Leitfähigkeit aufweist.
13. Strahlenschutzvorrichtung, dadurch gekennzeichnet, daß die Dipole (8, 9, 10, 11; 21, 22, 23) jeweils in Form einer Schlinge aus zwei gebogenen Haken (26, 27) gebildet sind.
14. Strahlenschutzvorrichtung, dadurch gekennzeichnet, daß zwischen den einander gegenüberliegenden Enden (29, 30) der Schlinge ein die Haken (26, 27) verbindender Steg (28) verläuft.
15. Strahlenschutzvorrichtung, dadurch gekennzeichnet, daß die Dipole (8, 9, 10, 11; 21, 22, 23) aus einer Kupferlitze mit kreisförmigem oder quadratischem Querschnitt bestehen.
16. Strahlenschutzvorrichtung, dadurch gekennzeichnet, daß der Abstand zwischen den Enden (29, 30) der Haken (26, 27) und dem Verbindungsteg (28) jeweils ein Mehrfaches der Materialstärke der Dipole (22) beträgt (Fig. 2).
17. Strahlenschutzvorrichtung, dadurch gekennzeichnet, daß ein oder mehrere Dipole (8, 9, 10, 11; 21, 22, 23) ganz oder teilweise mit einem Baumwollgewebe (32) und/oder einem Zellstoffgewebe (33) ein- oder mehrfach ummantelt sind (Fig.3 und 4).
18. Strahlenschutzvorrichtung, daß das Baumwollgewebe (32) und/oder das Zellstoffgewebe (33) in einer oder mehreren Lagen um das schwingende Gebilde (21) gewickelt ist (Fig. 3).
19. Strahlenschutzvorrichtung, daß das Baumwollgewebe (32) und das Zellstoffgewebe (33) jeweils als Schlauch ausgebildet ist, der über das schwingende Gebilde (23) gezogen ist.
20. Strahlenschutzvorrichtung, dadurch gekennzeichnet, daß die Enden (29, 30) der mit Baumwollgewebe (32) und/oder Zellstoffgewebe (33) ummantelten Haken (26, 27) der Dipole (22) etwa in einer der Materialstärke entsprechenden Länge freigehalten sind.
21. Strahlenschutzvorrichtung, dadurch gekennzeichnet, daß die elektromagnetisch schwingenden Gebilde (8, 9, 10, 11; 21, 22, 23) auf einer Schaumstoffunterlage, einer Kunststoff-Folie, einer Gummimatte oder dgl. als Trägermittel (24) aufgeklebt sind.
22. Strahlenschutzvorrichtung, dadurch gekennzeichnet, daß die Enden (29, 30) der Haken (26,
27) ein oder beidseitig durch ein Klebeband (28) fixiert sind.
23. Strahlenschutzvorrichtung, dadurch gekennzeichnet, daß die elektromagnetisch schwingenden Gebilde (8, 9, 10, 11; 21, 22, 23) zwischen dem Trägermittel (24) und einer Auflage (25) angeordnet sind.
24. Strahlenschutzvorrichtung, daß das Trägermittel (24) und/oder die Auflage (25) aus einem nicht leitenden Material besteht.
25. Strahlenschutzvorrichtung, dadurch gekennzeichnet, daß das Trägermittel (24) und/oder die Auflage (25) aus einem Schaumstoffmaterial, einer Kunststoff-Folie und/oder einer Gummimatte oder dgl. besteht.
26. Strahlenschutzvorrichtung, dadurch gekennzeichnet, daß die elektromagnetisch schwingenden Gebilde (8, 9, 10, 11; 21, 22, 23) auf dem Trägermittel (24) gleichmäßig verteilt angeordnet sind.
27. Strahlenschutzvorrichtung, dadurch gekennzeichnet, daß der seitliche Abstand zwischen den elektromagnetisch schwingenden Gebilden (8, 9, 10, 11; 21, 22, 23) und den Außenkanten des Trägermittels (24) etwa gleich groß ist.
28. Strahlenschutzvorrichtung, dadurch gekennzeichnet, daß jedes Grundteil (2, 3, 4, 5) jeweils ein elektromagnetisch schwingendes Gebilde (8, 9, 10, 11) aufweist, welches bezüglich der aufeinander senkrecht stehenden Mittelachsen des Grundteils (2, 3, 4, 5) im wesentlichen symmetrisch ausgebildet ist.

## Patentansprüche

1. Strahlenschutzvorrichtung gekennzeichnet durch wenigstens ein elektromagnetisch schwingendes Gebilde (2, 3, 4, 5; 21, 22, 23).

2. Strahlenschutzvorichtung nach Anspruch 1, gekennzeichnet durch die Verwendung als Schlaf-, Liege-, Sitz- und/oder Bodenmatte bzw. -belag.

3. Strahlenschutzvorrichtung nach Anspruch 1 oder 2, dadurch gekennzeichnet, daß die Strahlenschutzvorrichtung (1) aus wenigstens zwei Grundteilen (2, 3, 4, 5) zusammengesetzt ist, die jeweils wenigstens ein schwingendes Gebilde (8, 9, 10, 11) aufweisen (Fig. 1).

4. Strahlenschutzvorrichtung nach Anspruch 3, dadruch gekennzeichnet, daß die Grundteile (2, 3, 4, 5) Klettverschlüsse (6) aufweisen (Fig. 1).

5. Strahlenschutzvorrichtung nach Anspruch 3 oder 4, dadurch gekennzeichnet, daß die Grundteile (2, 3, 4, 5) Druckknöpfe aufweisen.

6. Strahlenschutzvorrichtgung nach einem der Ansprüche 3 bis 5, dadurch gekennzeichnet, daß jedes Grundteil ein Rechteck mit einer langen Seitenkante (a) von 800 bis 1200 mm und einer kurzen Seitenkante (b) von 400 bis 600 mm ist.

7. Strahlenschutzvorrichtung nach einem der Ansprüche 3 bis 6, dadurch gekennzeichnet, daß für einen Arbeitsplatz ein Grundteil (2) vorgesehen ist.

8. Strahlenschutzvorrichtung nach einem der Ansprüche 3 bis 6, dadurch gekennzeichnet, daß für einen Sitzplatz zwei Grundteile (3, 4) vorgesehen sind.

9. Strahlenschutzvorrichtung nach einem der Ansprüche 3 bis 6, dadurch gekennzeichnet, daß für einen Bettenplatz drei oder vier Grundteile (2, 3, 4, 5) vorgesehen sind.

10. Strahlenschutzvorrichtung nach einem der vorhergehenden Ansprüche, dadurch gekennzeichnet, daß das schwingende Gebilde (8, 9, 10, 11; 21, 22, 23) ein Dipol ist.
